# EUROPEAN PATENT APPLICATION

(11) **EP 2 721 993 A1**
(43) Date of publication of application: **23.04.2014**
(21) Application number: 12852916.1
(22) Date of filing: 07.08.2012
(51) Int. Cl.: A61B 1/06, G02B 23/24

(54) **ENDOSCOPE**

(30) Priority: 29.11.2011 JP 2011260774
(71) Applicant: OLYMPUS MEDICAL SYSTEMS CORP., Tokyo 151-0072 (JP)
(72) Inventor: SAKATA Hajime, Tokyo 151-0072 (JP)
(74) Representative: Schicker, Silvia
(86) International application number: PCT/JP2012/070034
(87) International publication number: WO 2013/080608

(57) **Abstract**

An endoscope 2 includes: a circular tube-shaped frame 45 disposed on a wiring route of a cable 58, the frame 45 allowing the cable 58 to be inserted therethrough; and a hole 48 formed in a barrel portion of the frame 45, the hole 48 allowing the cable 58 to be inserted therethrough. An excess of the cable 58 is drawn out from the hole 48 and the excess is wound and stored on the barrel portion of the frame 45, enabling the excess of the cable that transmits a signal to be effortlessly stored without an increase in the number of components.

## Description

### Technical Field

The present invention relates to an endoscope including a cable.

### Background Art

Some types of conventional endoscopes include endoscope connectors integrated with scope cables. In these endoscopes, an image pickup cable that transmits an image pickup signal from an image pickup unit is connected from an insertion portion to a video connector of an endoscope connector connected to a video processor, via an operation portion, a universal cable, which is a composite cable, and a light source connector of the endoscope connector, and a scope cable. Also, a switching cable that transmits a switching signal from the operation portion is connected from the operation portion to the video connector through the universal cable, the light source connector of the endoscope connector, and the scope cable. These cables that transmit electrical signals are provided with excesses for repair in consideration of repair workability, and in order to, e.g., route such cable excesses and/or avoid stress being imposed on other incorporated objects, as disclosed in, for example, Japanese Patent Application Laid-Open Publication No. 2001-252245, a portion partway of each cable excess is circularly wound and stored in a cable storage portion.

However, structures of the conventional endoscopes require blind work of winding a cable excess that transmits an electrical signal on a bobbin, which is a new component, and pressing the cable excess into a cable storage portion, and when the bobbin is assembled or the cable storage portion is closed during assembly, the cable excess may be caught in, e.g., an exterior case, resulting in, e.g., breaking or damage of the cable excess. Also, the bobbin has a fixed diameter and a length of the cable excess wound on the bobbin is determined by the diameter of the bobbin. In other words, in some cases, a length of a part of the cable resulting from a necessary number of turns of the cable being unwound from the bobbin and a length of a part of the cable necessary for, e.g., a repair do not correspond to each other. In such cases, a length of the cable corresponding to the difference value becomes loose, and when the cable storage portion is closed during assembly, this loose cable may be caught in, e.g., the exterior case, resulting in, e.g., breaking or damage of the cable.

Therefore, the present invention has been made in view of the above circumstances, and an object of the present invention is to provide an endoscope that enables an excess of a cable that transmits a signal to be effortlessly stored without an increase in the number of components.

### Disclosure of Invention

### Means for Solving the Problem

An endoscope according an aspect of the present invention includes: a tubular frame disposed on a wiring route of a cable, the frame allowing the cable to be inserted therethrough; and a hole formed in a barrel portion of the frame, the hole allowing the cable to be inserted therethrough. An excess of the cable is drawn out from the hole and the excess is wound and stored on the barrel portion of the frame.

### Brief Description of the Drawings

Fig. 1 is a perspective view illustrating an overall configuration of an endoscope apparatus;
Fig. 2 is a perspective view illustrating a peripheral and endoscope connectors;
Fig. 3 is a perspective view illustrating a configuration of a light source plug;
Fig. 4 is a cross-sectional view illustrating the configuration of the light source plug;
Fig. 5 is a perspective view illustrating a state before an endoscope 2 is held over a tag reading section of a cleaning/disinfecting apparatus;
Fig. 6 is a perspective view illustrating a state in which the endoscope 2 is held over the tag reading section of the cleaning/disinfecting apparatus;
Fig. 7 is a perspective view illustrating a configuration of an electric plug;
Fig. 8 is an exploded perspective view illustrating the configuration of the electric plug;
Fig. 9 is a perspective view illustrating a state before an electric cable is wound and stored on a connector frame;
Fig. 10 is a perspective view illustrating a state in which the electric cable is wound and stored on the connector frame;
Fig. 11 is a cross-sectional view illustrating a state in which the electric cable is wound and stored on the connector frame;
Fig. 12 is a cross-sectional view illustrating a state in which a connector cover, a reinforcing tube and a bend preventing portion are assembled to the connector frame;
Fig. 13 is a plan view illustrating a configuration of a long hole according to a modification;
Fig. 14 is a cross-sectional view illustrating a configuration of a long hole according to a modification;
Fig. 15 is a cross-sectional view of a connector frame with a cover body provided thereto;
Fig. 16 is a cross-sectional view illustrating an attachment configuration of an earth terminal provided at a connector case of a light source plug according to a first reference example;
Fig. 17 is a perspective view illustrating the connector case and a metal frame of the light source plug provided with a backlash prevention plate at a part to which the earth terminal is to be attached;
Fig. 18 is a perspective view illustrating the connector case and the metal frame of the light source plug provided with stoppers on the backlash prevention plate;
Fig. 19 is a cross-sectional view illustrating an attachment configuration of a front water feeding opening fitting provided at a connector case of a light source plug according to a second reference example;
Fig. 20 is a perspective view illustrating a part of the connector case of the light source plug to which the front water feeding opening fitting is to be attached;
Fig. 21 is a perspective view illustrating the connector case of the light source plug provided with a backlash prevention plate at the part to which the front water feeding opening fitting is to be attached;
Fig. 22 is a perspective view illustrating the connector case of the light source plug provided with stoppers on the backlash prevention plate;
Fig. 23 is a perspective view illustrating an air/water feeding block according to a third reference example;
Fig. 24 is a cross-sectional view of a light source plug, which illustrates an attachment configuration of a water feeding opening fitting and an air feeding opening fitting provided at a connector case and the air/water feeding block provided inside;
Fig. 25 is a transverse sectional diagram illustrating an attachment configuration of the water feeding opening fitting and the air feeding opening fitting provided at the connector case and the air/water feeding block provided inside; and
Fig. 26 is a vertical sectional diagram illustrating an attachment configuration of the water feeding opening fitting and the air feeding opening fitting provided at the connector case and the air/water feeding block provided inside.

### Best Mode for Carrying Out the Invention

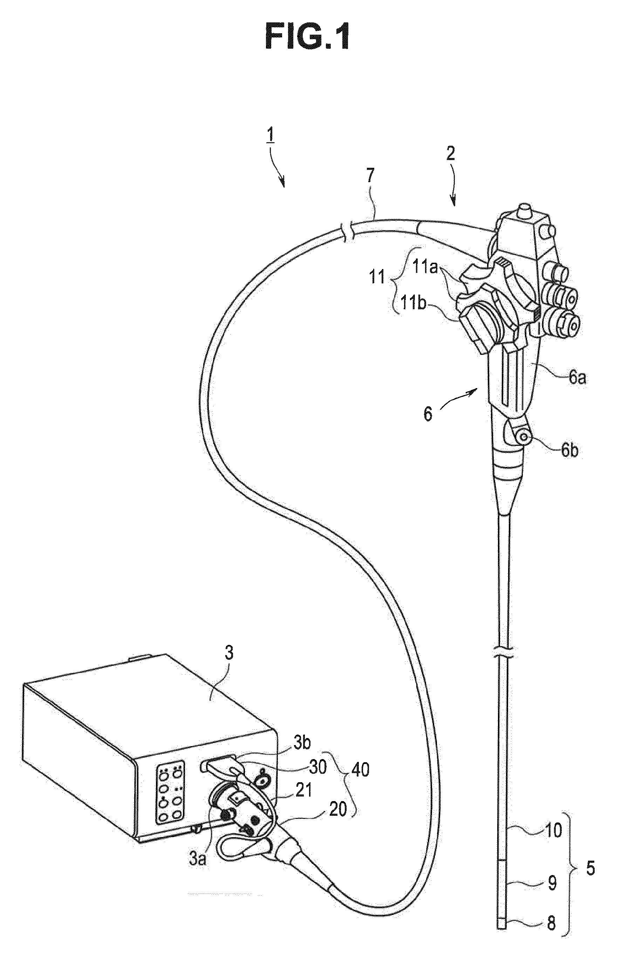

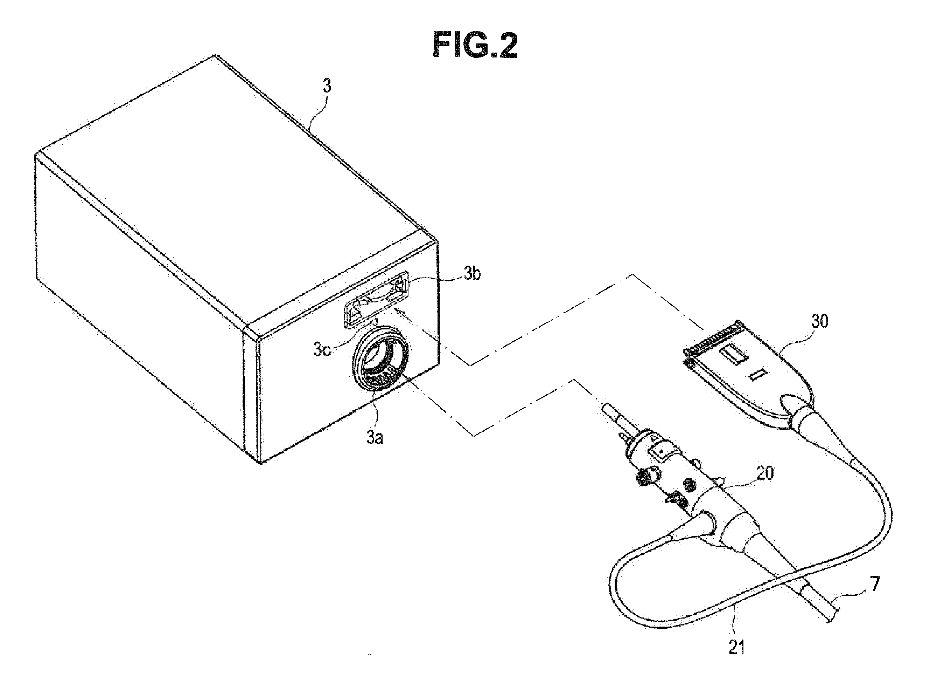

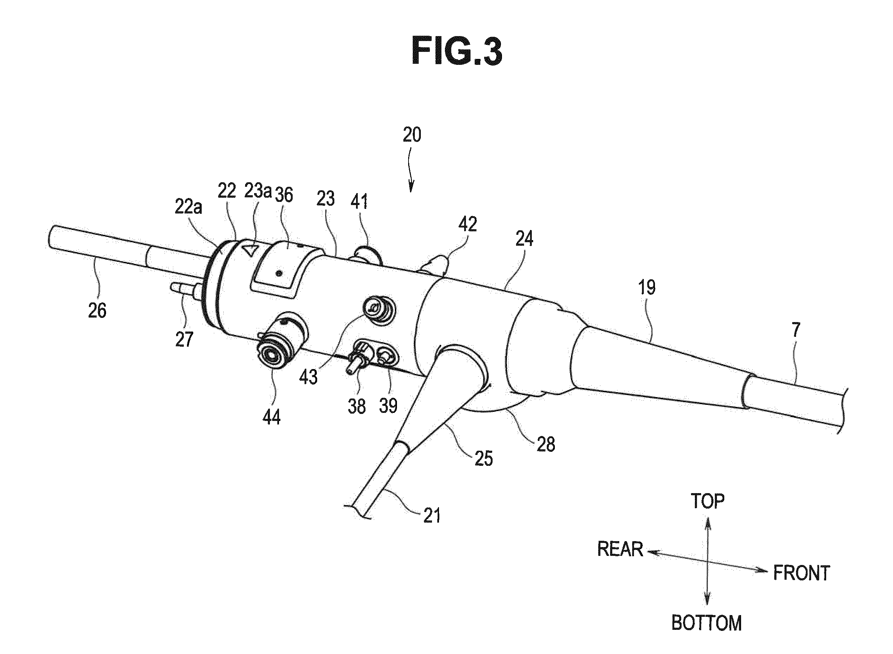

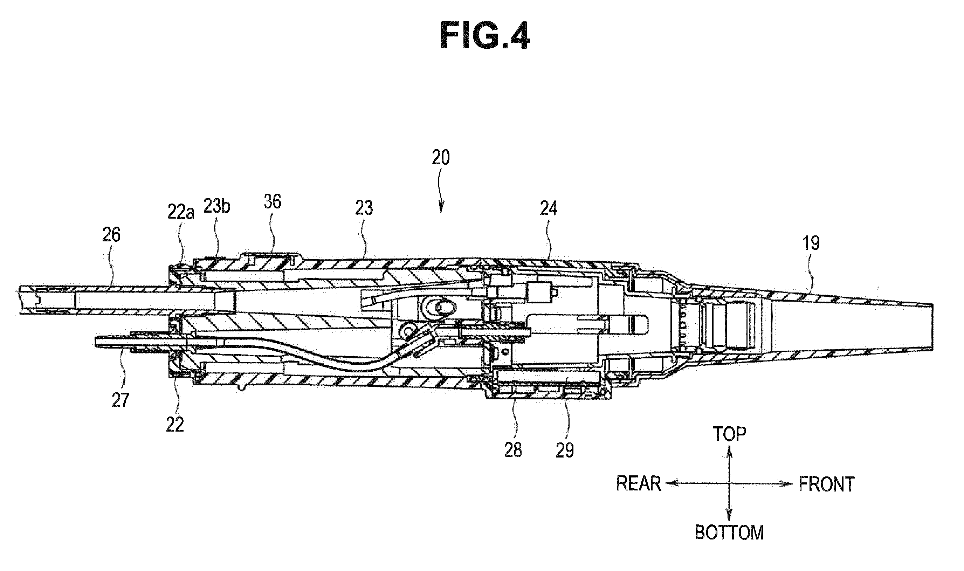

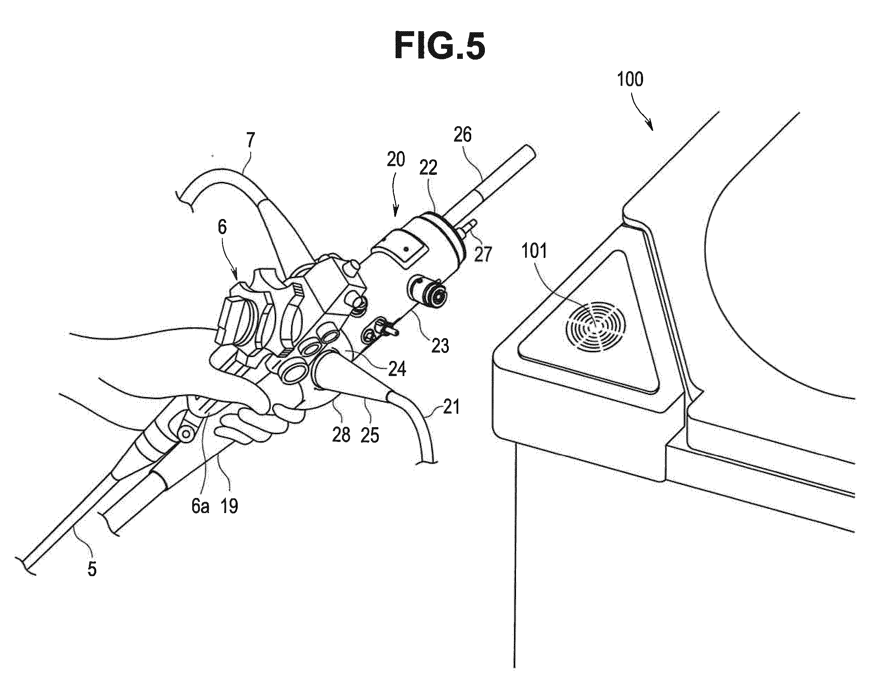

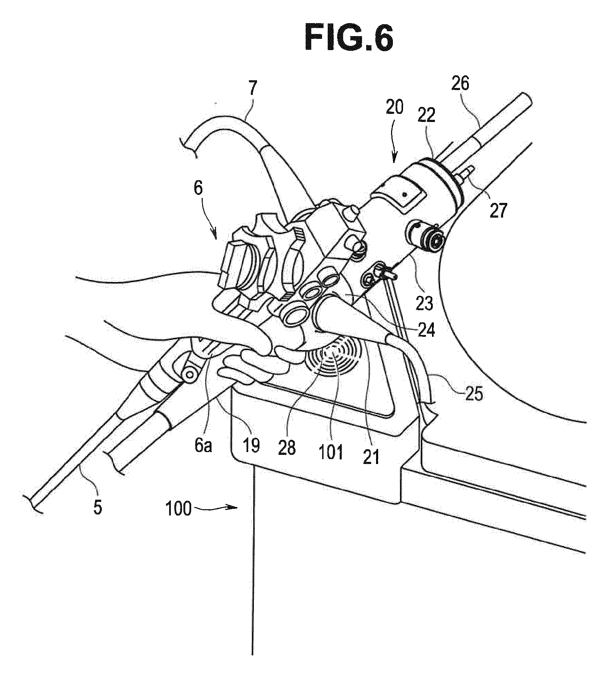

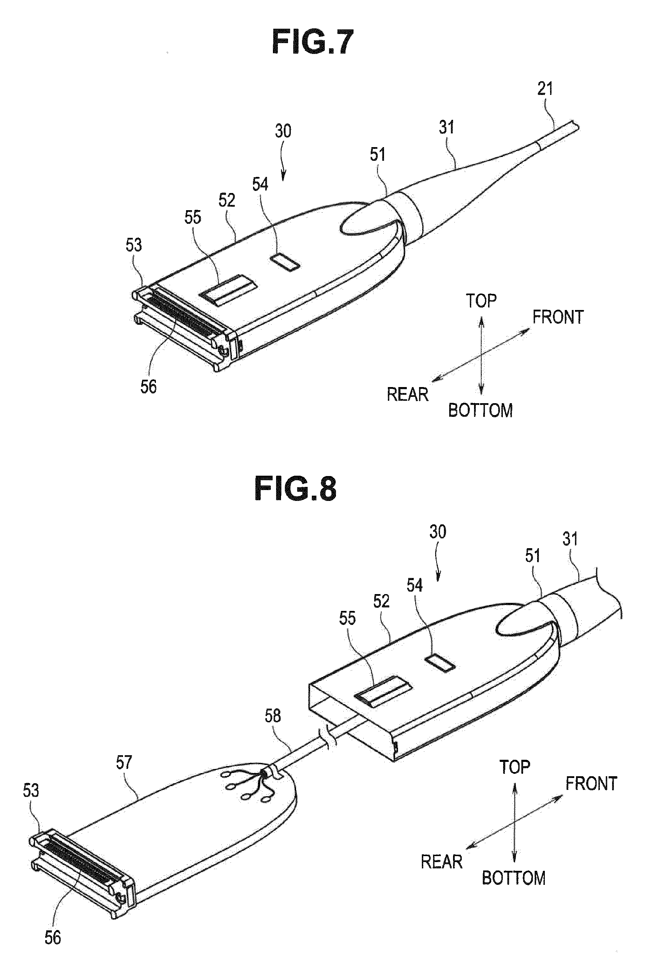

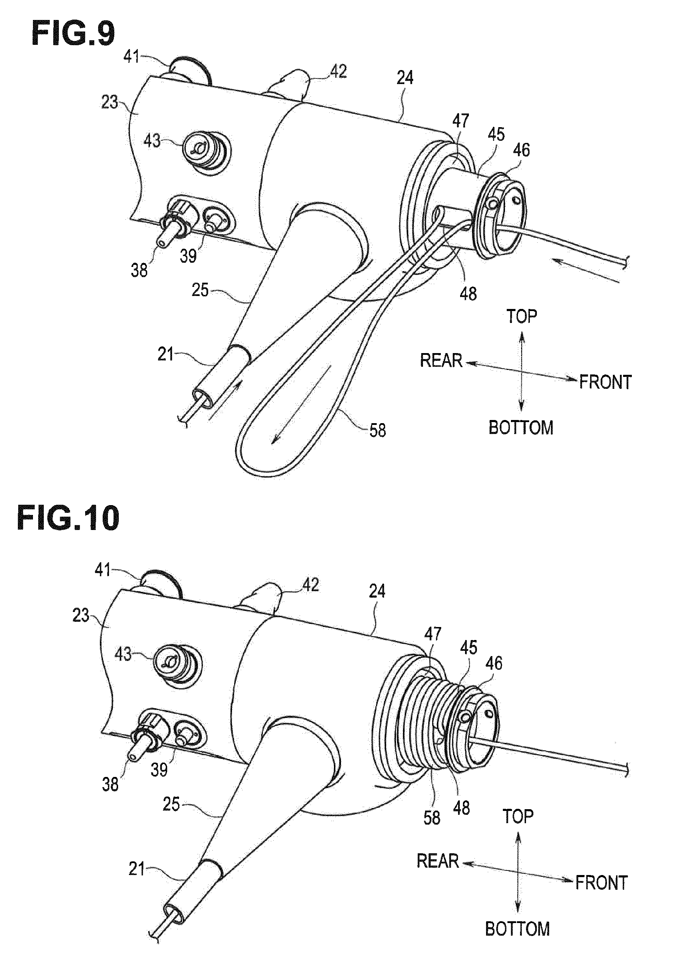

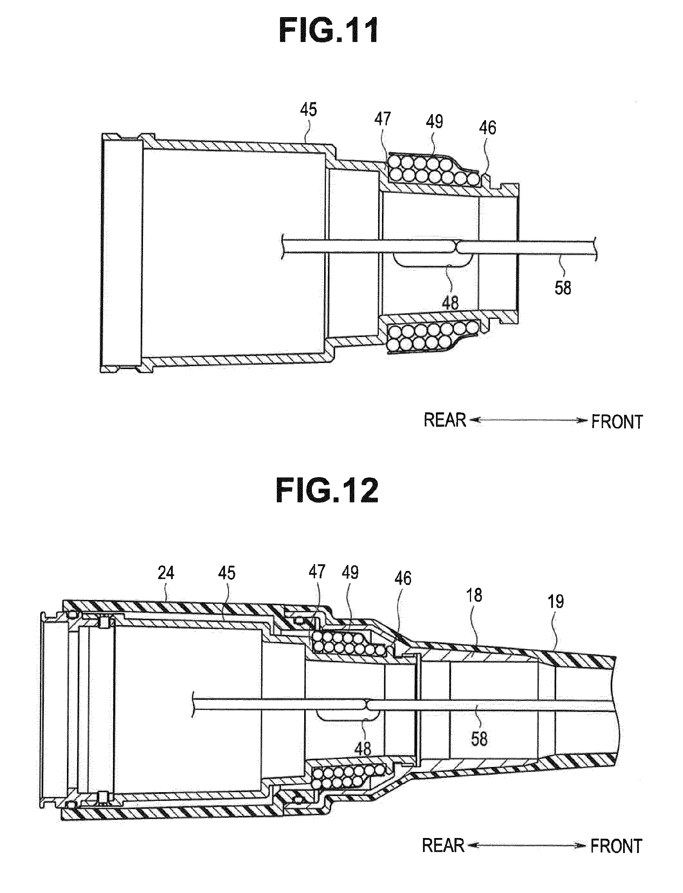

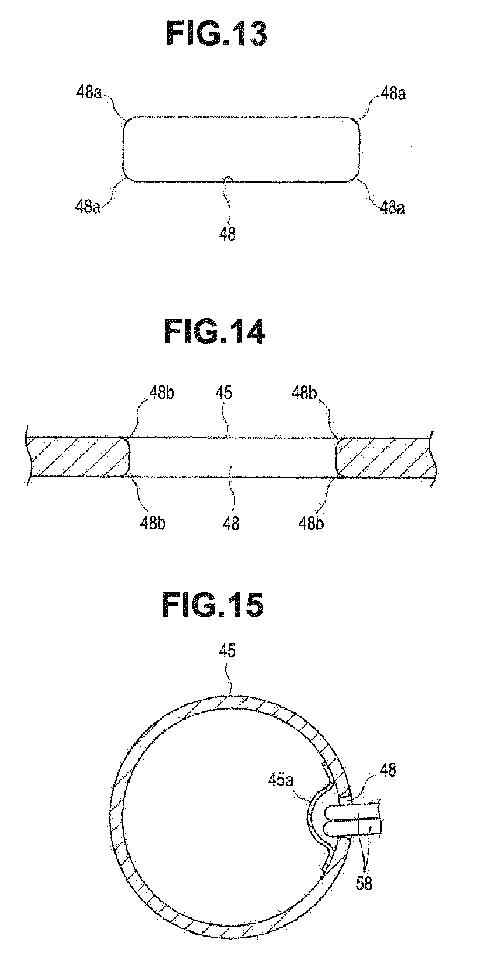

An embodiment of the present invention will be described below with reference to the drawings. Figs. 1 to 15 relate to an aspect of the present invention: Fig. 1 is a perspective view illustrating an overall configuration of an endoscope apparatus; Fig. 2 is a perspective view illustrating a peripheral and endoscope connectors; Fig. 3 is a perspective view illustrating a configuration of a light source plug; Fig. 4 is a cross-sectional view illustrating the configuration of the light source plug; Fig. 5 is a perspective view illustrating a state before an endoscope 2 is held over a tag reading section of a cleaning/disinfecting apparatus; Fig. 6 is a perspective view illustrating a state in which the endoscope 2 is held over the tag reading section of the cleaning/disinfecting apparatus; Fig. 7 is a perspective view illustrating a configuration of an electric plug; Fig. 8 is an exploded perspective view illustrating the configuration of the electric plug; Fig. 9 is a perspective view illustrating a state before an electric cable is wound and stored on a connector frame; Fig. 10 is a perspective view illustrating a state in which the electric cable is wound and stored on the connector frame; Fig. 11 is a cross-sectional view illustrating a state in which the electric cable is wound and stored on the connector frame; Fig. 12 is a cross-sectional view illustrating a state in which a connector cover, a reinforcing tube and a bend preventing portion are assembled to the connector frame; Fig. 13 is a plan view illustrating a configuration of a long hole according to a modification; Fig. 14 is a cross-sectional view illustrating a configuration of a long hole according to a modification; and Fig. 15 is a cross-sectional view of a connector frame with a cover body provided thereto.

Fig. 1 illustrates an endoscope apparatus 1 according to the present invention. The endoscope apparatus 1 includes an endoscope 2, a processing apparatus (camera control unit: hereinafter referred to as "CCU") 3, which is a peripheral connected to the endoscope 2, the peripheral serving as a video processor that performs various types of signal processing and doubling as a light source apparatus that incorporates a light source for illuminating light, and a non-illustrated monitor that receives a signal outputted from the CCU 3 and displays, e.g., an image of a site to be observed.

The endoscope 2 includes an insertion portion 5, which is an elongated hollow member to be inserted into a site to be observed, an operation portion 6 including a grasping portion 6a provided so as to be continuous with a proximal end portion of the insertion portion 5, and a universal cable 7 provided so as to extend from a side face of the operation portion 6. The insertion portion 5 includes a distal end portion 8 that incorporates, e.g., an illumination system and an image pickup system on a distal end side, and a bending portion 9, which is a bendable movable portion, is continuous with a rear portion of the distal end portion 8.

Furthermore, a flexible tube portion 10 having a long length and flexibility and including a flexible tubular member, is provided so as to be continuous with a rear portion of the bending portion 9. Also, at the operation portion 6, e.g., a bending operation portion 11 including a bending operation knob 11a for performing a bending operation of the bending portion 9 and a fixing lever 11b for fixing the bending operation knob 11a at a desired rotational position is disposed in a predetermined manner. Here, reference numeral 6b denotes a treatment instrument insertion port, which is in communication with a proximal end of a treatment instrument insertion channel (not illustrated) that communicates with the insertion portion 5.

The endoscope 2 includes endoscope connectors 40 at an end portion of the universal cable 7, and the endoscope connectors 40 include a light source plug 20, which is a first endoscope connector, and an electric plug 30, which is a second endoscope connector connected to the light source plug 20 via a connector cable 21. The endoscope 2 and the CCU 3 are connected as a result of male connectors of the light source plug 20 and the electric plug 30 engaging with respective female connectors provided at the CCU 3, which are a light source connector 3a and a receptacle connector 3b.

In the present embodiment, the light source plug 20 and the light source connector 3a are cylindrical connectors, and the light source plug 20 is detachably fitted in the light source connector 3 a to bring respective contact points into mechanical contact with each other, enabling provision of an illuminating light transmission channel and electrical connection. Also, in the present embodiment, the electric plug 30 and the receptacle connector 3b are flat connectors, and the electric plug 30 is detachably fitted in the receptacle connector 3b to bring respective contact points into mechanical contact with each other, enabling provision of electrical connection.

Note that the light source connector 3a or the receptacle connector 3b is disposed in such a manner that an opening portion for inserting/fitting the light source plug 20 or the electric plug 30 therein is exposed at one surface (front face portion) of a housing of the CCU 3. Each of the light source connector 3a and the receptacle connector 3b includes a locking mechanism portion that holds or cancels a fitted state of the light source plug 20 and the electric plug 30 connected thereto. Also, here, although the CCU 3 is provided as a peripheral, a configuration in which a light source apparatus and a video processor are provided as separate peripherals and the light source connector 3a and the receptacle connector 3b are connected to respective apparatuses may be employed.

Next, a configuration of the light source plug 20, which is one of the endoscope connectors 40 included in the endoscope 2 according to the present embodiment, will be described below with reference to the drawings in Figs. 2 and 3. Note that the light source plug 20 according to the present embodiment is disposed at a proximal end of the endoscope 2, which is a terminal end of the universal cable 7, and the below description is provided assuming that a side of the light source plug 20 connected to the universal cable 7 is a distal end (front side) and a side of the light source plug 20 connected to a peripheral (here, the CCU 3) is a proximal end (rear side). Furthermore, since the light source plug 20 is connected to the CCU 3 placed in an operating room, the below description is provided assuming that upper, lower, left and right directions are based on directions when the light source plug 20 is connected to the CCU 3.

First, as illustrated in Figs. 2 and 3, an exterior of the light source plug 20 is formed mainly by a connection plug portion 22 that is formed in a substantially disc-like shape and includes an engagement member 22a provided at an outer circumferential portion for engagement with the light source connector 3 a, which is a C ring or the like, a connector case 23, which is a substantially cylindrical case body provided so as to be continuous with the connection plug portion 22, and a connector cover 24, which is a tubular case body and serves as a grasping portion to be grasped by a user mainly with his/her right hand, in this order from the rear side.

From an end face (rear end face) of the connection plug portion 22, a light guide opening fitting 26 that receives illuminating light from the peripheral extends on the upper side and an air feeding opening fitting 27 that feeds a gas from the peripheral extends on the lower side.

At one side face portion of the connector case 23, a water feeding opening fitting 38, an air feeding opening fitting 39 and a water leakage detection opening fitting 44 are provided. Furthermore, at a part partway of an upper side of an outer circumferential portion of the connector case 23, a nameplate 36 for indicating, e.g., a manufacturer, a model, a date of manufacture and usage are provided, and at a front part on the upper side of the connector case 23, an earth terminal 43 is provided.

Note that as mentioned above, the upper and lower directions of the light source plug 20 are those where the light source plug 20 is connected to the peripheral, and here, the side on which the nameplate 36 provided on the connector case 23 is provided is the upper side, and the nameplate 36 serves as an indicator indicating an upper portion of the light source plug 20.

Furthermore, an indicator portion 23a having a triangular shape here is provided at a rear upper portion of the connector case 23. The indicator portion 23a serves as an indicator indicating the upper portion of the light source plug 20 together with the nameplate 36, and also serves as an indicator that prescribes a direction of connection of the light source plug 20 to the light source connector 3 a by aligning a tip on the rear side of the indicator portion 23a with an indicator portion 3c having a triangular shape here, the indicator portion 3c being provided above the light source connector 3a at a front face portion (front panel) of the CCU 3. Note that the shape of the indicator portion 23a is not limited to a triangular shape and may be any shape such as a circular shape or a quadrangular shape.

Also, a bend preventing portion 25, from which the connector cable 21 extends, the bend preventing portion 25 covering an extension root part of the connector cable 21, is connected to a side face of the connector cover 24.

The light source plug 20 includes a tag-incorporated portion 28, which is an individual identification portion formed so as to project at a lower portion of an outer surface covered by the connector cover 24. In the tag-incorporated portion 28, a plate-like RFID chip 29, which is a model information storage tag, is disposed at a lower position inside the connector cover 24 in such a manner that a plate surface is parallel to a horizontal direction perpendicular to the upper and lower directions (see Fig. 4). Note that an exterior portion forming the tag-incorporated portion 28 of the connector cover 24 has a substantially cylindrical shape and a lower portion thereof has a flat face so as to be parallel with the plate surface of the RFID chip 29. Note that in the RFID chip 29, various pieces of information on the endoscope 2 such as model information, a serial number and records of cleaning/disinfecting by the later-described endoscope cleaning/disinfecting apparatus are stored.

After use, the endoscope 2 is set in a cleaning bath of an endoscope cleaning/disinfecting apparatus 100 (see Figs. 5 and 6), which is an external apparatus, for cleaning/disinfecting. Here, first, the endoscope connectors 40 are pulled out from the CCU 3. A user pulls the light source plug 20 and the electric plug 30 of the endoscope connectors 40 from the light source connector 3a or the receptacle connector 3b of the CCU 3, respectively.

Since the insertion portion 5 has a long length and is flexible, and also in order to prevent damage of the illumination system and the image pickup system incorporated in the distal end portion 8 and further in order to prevent damage of an exterior of the insertion portion 5 as a result of projection portions of the endoscope connectors 40 coming into contact with the exterior, when a user grasps the endoscope 2, in many cases, as illustrated in Figs. 5 and 6, the user grasps the insertion portion 5 with one of his/her hands and grasps both the operation portion 6 and the light source plug 20 of the endoscope connectors 40 with the other hand, here, the left hand. Then, the user carries the endoscope 2 grasped with both hands to the endoscope cleaning/disinfecting apparatus 100 and holds the tag-incorporated portion 28 with the RFID chip 29 incorporated therein, which is provided at a lower portion of the light source plug 20 over a tag reading section 101 provided at an upper corner portion of the endoscope cleaning/disinfecting apparatus 100.

Here, the endoscope 2 according to the present embodiment has a configuration in which the RFID chip 29 is provided in the lower portion of the light source plug 20 of the endoscope connectors 40, enabling the tag-incorporated portion 28 with the RFID chip 29 incorporated therein to be easily held over the tag reading section 101 of the endoscope cleaning/disinfecting apparatus 100 with a position of the light source plug 20 pulled out from the CCU 3 unchanged. In other words, before the user sets the endoscope 2 in the cleaning bath of the endoscope cleaning/disinfecting apparatus 100 after pulling the endoscope 2 out from the CCU 3, the user can simply hold the tag-incorporated portion 28 over the tag reading section 101 of the endoscope cleaning/disinfecting apparatus 100 with positions of the operation portion 6 and the light source plug 20 of the endoscope connectors 40 grasped by his/her left hand unchanged, i.e., the vertical direction thereof maintained, without turning or twisting the wrist to make the endoscope cleaning/disinfecting apparatus 100 read, e.g., the model information.

In other words, the vertical direction of the light source plug 20 of the endoscope 2 is prescribed. More specifically, the vertical direction when the light source plug 20 is connected to the light source connector 3a of the CCU 3 is prescribed so that the nameplate 36 and the indicator portion 23 a provided at the connector case 23 are on the upper side. The RFID chip 29 is disposed inside the tag-incorporated portion 28 of the connector cover 24, the tag-incorporated portion 28 being disposed at a position on the opposite side of the nameplate 36 and the indicator portion 23a (lower position rotated by 180° along an outer circumference of the connector cover 24 relative to the nameplate 36 and at the upper positions).

Since the lower portion of the tag-incorporated portion 28 of the light source plug 20 has a flat surface that is parallel to the plate surface of the RFID chip 29, the tag-incorporated portion 28 is held over a reading surface of the tag reading section 101 of the endoscope cleaning/disinfecting apparatus 100 in substantially parallel to the reading surface, and thus, the RFID chip 29 and a reader (not illustrated) incorporated in the tag reading section 101 become substantially parallel to each other, which suppresses occurrence of reading errors and ensures reliable reading of the endoscope information by the RFID reader. In other words, the tag reading section 101 with the RFID reader incorporated therein, the RFID reader reading the information in the RFID chip 29, is provided, here, at a position in an upper face of the endoscope cleaning/disinfecting apparatus 100, enabling the RFID chip 29 and the RFID reader of the tag reading section 101 to be parallel and close to each other, ensuring reliable reading.

Note that the RFID chip 29 in the light source plug 20 is not limited to one having the above configuration, and may be provided at any position in the light source plug 20 according to the position of the tag reading section 101 provided in the endoscope cleaning/disinfecting apparatus 100. In other words, the RFID chip 29 may be provided at any position in the light source plug 20 as long as such position is a position that enables the RFID chip 29 and the RFID reader to be parallel and close to each other with the position of the light source plug 20 pulled out from the light source connector 3 a of the CCU 3 unchanged without turning or twisting the wrist when the user holds the tag-incorporated portion 28 of the light source plug 20 over the tag reading section 101 with the RFID reader incorporated therein in the endoscope cleaning/disinfecting apparatus 100.

Also, the RFID chip 29 is not limited to one having the above-described configuration and may be provided in a connector connected to a peripheral, for example, the electric plug 30.

Furthermore, although the configuration of the RFID chip 29 and the RFID reader has been described above, the present invention is not limited to thereto, and a configuration in which a transponder, a bar code or the like is provided on the tag-incorporated portion 28 side of the endoscope 2 and a transponder reader, a bar code reader or the like is provided on the tag reading section 101 side of the endoscope cleaning/disinfecting apparatus 100 may be employed.

Next, a configuration of the electric plug 30, which is one of the endoscope connectors 40 included in the endoscope apparatus 1 according to the present embodiment, will be described below with reference to the drawings in Figs. 7 and 8. Note that the electric plug 30 according to the present embodiment is also disposed at the proximal end of the endoscope 2, which is a terminal end of the connector cable 21, and the below description is provided assuming that the side of the electric plug 30 connected to the connector cable 21 is a distal end (front side) and the side of the electric plug 30 connected to a peripheral (here, the CCU 3) is a proximal end (rear side). Furthermore, since the electric plug 30 is also connected to the CCU 3 placed in an operating room, the below description is provided assuming that upper, lower, left and right directions are based on directions when the electric plug 30 is connected to the CCU 3.

For details, the electric plug 30 has the outer appearance shape and the contact structure illustrated in Figs. 7 and 8.

The electric plug 30 includes a flat exterior case 52 provided at a root portion of the connector cable 21, the exterior case 52 integrally including a cylindrical sleeve 51 to which a bend preventing portion 31 is connected, and a terminal portion 53 that projects from a rear end of the exterior case 52. From among the exterior case 52 and the terminal portion 53, at least the terminal portion 53 includes an insulating member of, e.g., a resin material.

A mark 54 such as "UP" for confirmation of the direction of the electric plug 30 when the electric plug 30 is inserted into the receptacle connector 3b is formed at a site on the sleeve 51 side of the exterior case 52. Behind the mark 54, an elongated projection portion 55 that engages with the locking mechanism portion of the receptacle connector 3b is provided. Also, a plurality of electric contacts 56 are provided at predetermined intervals in the terminal portion 53.

A substrate 57 housed in the exterior case 52 is connected to a front side of the terminal portion 53, and an electric cable 58 including a bundle of a plurality of signal wires connected to the substrate 57 extends from the substrate 57. The electric cable 58 is inserted through the inside of the connector cable 21, and an end portion of the electric cable 58 opposite to an end portion connected to the substrate 57 is connected to an image pickup apparatus (not illustrated), which includes, e.g., a CCD or a CMOS, incorporated in the distal end portion 8 of the insertion portion 5. Note that the electric cable 58 has a predetermined length of a cable excess as a working length for assembly, repair and maintenance.

It is necessary that the cable excess of the electric cable 58 be housed in some part of the endoscope 2. Therefore, in the present embodiment, as illustrated in Figs. 9 to 12, a configuration is provided in which the electric cable 58 is wound and stored on a front end part of a metal connector frame 45, which is a cylindrical frame member formed so as to have a stepped structure, the connector frame 45 being housed in the connector cover 24 in a wiring route of the electric cable 58.

More specifically, the connector frame 45 includes a rib 46 having an outward-directed flange shape, a step portion 47 formed on the rear side of the rib 46, and a long hole 48 with both opposite ends formed in an circular arc shape, the long hole 48 being provided in a part of a barrel portion of the connector frame 45 that forms a bottom face of a circumferential groove provided in an outer circumference of the connector frame 45 between the rib 46 and the step portion 47, and the electric cable 58 is wound in the circumferential groove having a predetermined width, which is formed by the rib 46 and the step portion 47.

More specifically, the electric cable 58 connected to the image pickup apparatus is made to pass through the inside of each of the insertion portion 5, the operation portion 6 and the universal cable 7, is inserted from a front end opening portion of the connector frame 45, is made to extend out from a side portion of the connector cover 24 and is then connected to the substrate 57 of the terminal portion 53. Then, the cable excess of the electric cable 58 is drawn out in a loop from the long hole 48 of the connector frame 45, is folded back into two (see Fig. 9), and is then wound on the barrel portion between the rib 46 and the step portion 47 of the connector frame 45 (see Fig. 10). The wound cable excess of the electric cable 58 is fixed by a fixing member such as a tape 49 so as not to spread out, in order to avoid, e.g., raveling (see Fig. 11).

Subsequently, the connector cover 24 is attached to the connector frame 45 and a metal reinforcing tube 18 is threadedly attached to the connector cover 24 so as to cover a front outer circumference of the connector cover 24. Lastly, a bend preventing portion 19, which is a cover for reducing concentration of stress from an external force, is attached to the reinforcing tube 18 so as to cover an outer circumferential portion of the reinforcing tube 18 (see Fig. 12). Note that the reinforcing tube 18 may be formed integrally with the bend preventing portion 19 by means of, e.g., insert molding.

Note that although each of the opposite ends of the long hole 48 of the connector frame 45 has a circular arc shape for prevention of damage of the electric cable 58 here, the present invention is not limited thereto, it is possible that each of corner portions 48a at four corners is rounded (see Fig. 13), an exact circular hole is provided or corner portions 48b at opening surfaces are chamfered (see Fig. 14). Also, although the long hole 48 is provided without protruding from the part of the barrel portion that forms the circumferential groove provided at the outer circumference of the connector frame 45, the present invention is not limited thereto, the long hole 48 may be a U-shaped cut extending from the part of the barrel portion that forms the circumferential groove to an end face of the connector frame 45, that is, extending from the end face on the universal cable 7 side of the connector frame 45 to the part of the barrel portion that forms the circumferential groove in a tube axis direction of the connector frame 45. Furthermore, as illustrated in Fig. 15, a cover body 45a for isolating the electric cable 58 from other incorporated components may be provided in an inner circumferential part of the connector frame 45 in which the long hole 48 is formed.

As described above, the endoscope 2 according to the present embodiment has a configuration in which the cable excess of the electric cable 58 for assembly, repair and maintenance has an arbitrary length and is wound and stored on the barrel portion of connector frame 45, which is conventionally provided, without an increase in the number of components. Furthermore, the front end part of the connector frame 45 is exposed during assembly of the endoscope 2, the work of storing the cable excess of the electric cable 58 can be viewed while the work is performed, which prevents the cable excess of the electric cable 58 from being caught in an exterior case such as the reinforcing tube 18 or the bend preventing portion 19, and thus enables prevention of, e.g., breakage and/or damage of the electric cable 58.

Note that although a configuration in which the cable excess of the electric cable 58 is wound and stored in the part of the barrel portion that provides the circumferential groove in the connector frame 45 on the universal cable 7 side of the connector frame 45 inside the connector cover 24 of the light source plug 20 has been indicated here, the present invention is not limited thereto, and for example, like a frame in an end portion on the connector cable 21 side of the light source plug 20, a frame in an end portion on the universal cable 7 side or the insertion portion 5 side of the operation portion 6 or a frame in an end portion on the connector cable 21 side of the electric plug 30, it is possible that a long hole is formed in a tubular frame body in an insertion route of the electric cable 58, and the cable excess of the electric cable 58 is wound and stored on a barrel portion of the frame body. Also, although the circumferential groove and the long hole 48 are provided in the circular tube-shaped connector frame 45 here, the present invention is not limited thereto, and any tubular member to which the electric cable 58 is to be inserted may be employed: for example, the long hole 48 from which the electric cable 58 is drawn out may be provided in a part of a barrel portion of a tubular member having a substantially quadrangular outer shape, the part forming a circumferential groove provided at an outer circumference of the tubular member, and the electric cable 58 may be wound on the part. Also, the number of cables drawn out from the long hole 48 and wound on the barrel portion is not limited to one, a plurality of cables may be wound. Furthermore, although the electric cable 58 connected to the image pickup apparatus has been described as an example here, the present invention is not limited thereto, an excess of any wire-like member disposed inside the endoscope 2, for example, a light guide cable, an electric cable connected to an LED light source for an LED light source-equipped endoscope, a fluid supply/suction tube or an electric cable connected to an ultrasound transducer of an ultrasound endoscope, may be wound and stored with the configuration described above.

### (Reference examples)

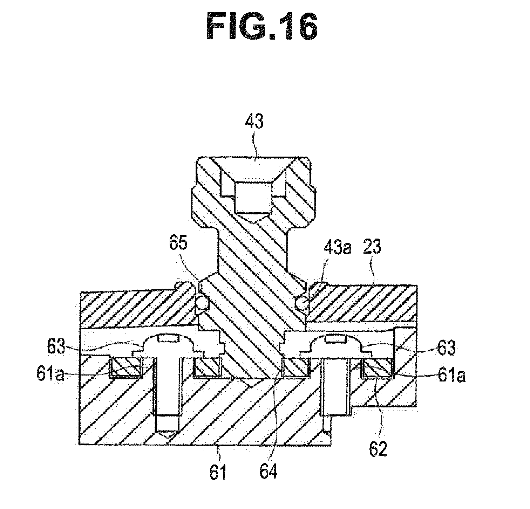

Next, reference examples of components included in the endoscope 2 according to the present application will be described below with reference to Figs. 16 to 24.

### (First reference example)

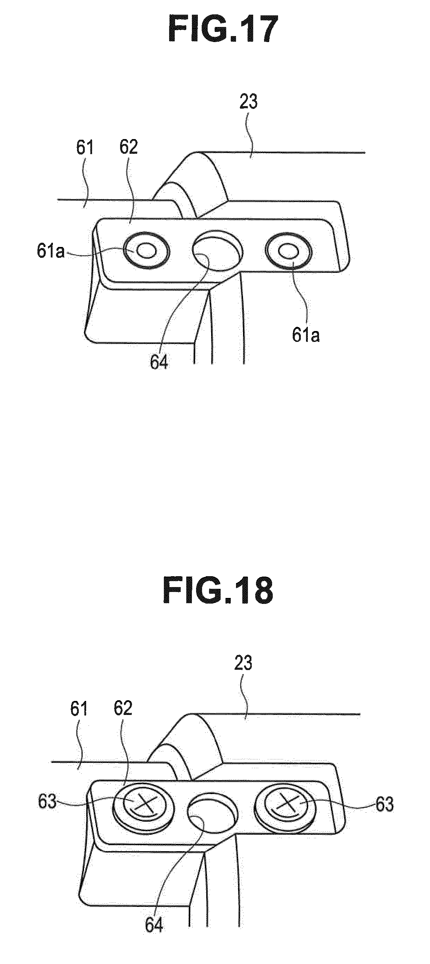

Figs. 16 to 18 relate to a first reference example: Fig. 16 is a cross-sectional view illustrating an attachment configuration of an earth terminal provided at a connector case of a light source plug; Fig. 17 is a perspective view illustrating the connector case and a metal frame of the light source plug provided with a backlash prevention plate at a part to which the earth terminal is to be attached; and Fig. 18 is a perspective view illustrating the connector case and the metal frame of the light source plug provided with stoppers on the backlash prevention plate.

As illustrated in Figs. 16 to 18, the earth terminal 43 is fixed so as to be electrically conductive in connection with a frame 61, which is a metal connector frame that serves as a stiffening member in the connector case 23. For the fixing, the frame 61 includes two projection portions 61a each including a screw hole formed therein, and two hole portions of a backlash prevention plate 62 are loosely fitted in the two projection portions 61a, respectively (see Fig. 17). Then, the backlash prevention plate 62 is attached to the frame 61 in such a manner that the backlash prevention plate 62 is movable within a predetermined range from the frame 61 but does not come off from the frame 61, as a result of two stoppers 63, which are, for example, flanged screws, being threadedly attached to the respective projection portions 61a, and thereby the backlash prevention plate 62 being brought into contact with the two stoppers 63. Note that the backlash prevention plate 62 includes a screw hole 64, which is a thread connection portion to which an end portion of the earth terminal 43 is threadedly connected.

The earth terminal 43 is inserted and fitted in a hole portion 65 of the connector case 23 and threadedly attached to the screw hole 64 of the backlash prevention plate 62. When the earth terminal 43 and the backlash prevention plate 62 are threadedly connected, the backlash prevention plate 62 loosely fitted in the frame 61 rises and hits the stoppers 63. Then, the earth terminal 43 is fixed so as to be electrically conductive in contact with the frame 61. Note that in the earth terminal 43, a seal 43a such as an O ring for holding airtightness when the earth terminal 43 is attached to the hole portion 65 of the connector case 23 is provided.

As described above, in the present reference example, even if manufacturing variations and/or deformations of components occur at a position of the hole portion 65 of the connector case 23, the backlash prevention plate 62 attached in a loosely fitted state to the frame 61 moves to a proper position, whereby misalignment between a center axis of the earth terminal 43 and a center axis of the hole portion 65 can be suppressed, ensuring reliable maintenance of the airtightness of the connector case 23 and enabling the earth terminal 43 to be stably fixed so as to be electrically conductive in contact with the frame 61.

### (Second reference example)

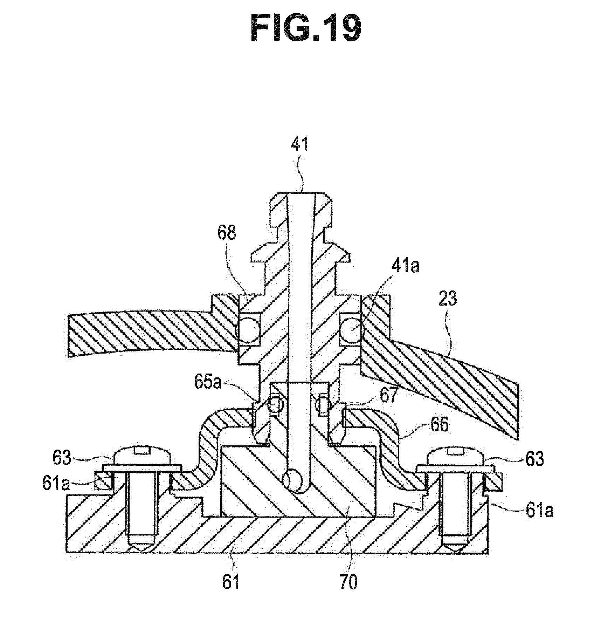

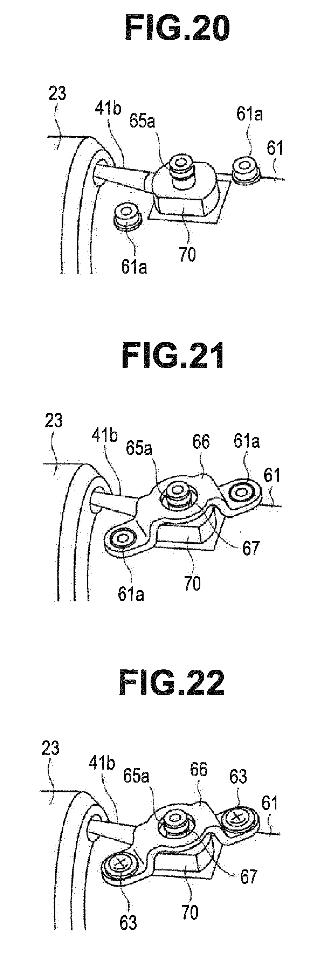

Figs. 19 to 22 relate to a second reference example: Fig. 19 is a cross-sectional view illustrating an attachment configuration of a front water feeding opening fitting provided at a connector case of a light source plug; Fig. 20 is a perspective view illustrating a part of the connector case of the light source plug to which the front water feeding opening fitting is to be attached; Fig. 21 is a perspective view illustrating the connector case of the light source plug provided with a backlash prevention plate at the part to which the front water feeding opening fitting is to be attached; Fig. 22 is a perspective view illustrating the connector case of the light source plug provided with stoppers on the backlash prevention plate.

As illustrated in Figs. 19 to 22, here, as in the first reference example, for attachment of the front water feeding opening fitting 41, the front water feeding opening fitting 41 is fixed via the frame 61, which is a reinforcing member, and a backlash prevention plate 66 inside the connector case 23. Note that the front water feeding opening fitting 41 is inserted and fitted in a conduit block 70 that is connected to a front water feeding conduit 41 b and installed on the frame 61.

For the fixing, first, the conduit block 70 connected to the front water feeding conduit 41b is placed at a predetermined position on the frame 61 (see Fig. 20). Then, the backlash prevention plate 66 is arranged so that two projection portions 6 1 a of the frame 61 are loosely fitted in two hole portions of the backlash prevention plate 66, respectively (see Fig. 21). Note that the backlash prevention plate 66 here is a plate member having a hat-like shape in cross-section so as not to come into contact with the conduit block 70. Then, the backlash prevention plate 66 is also attached to the frame 61 in such a manner that the backlash prevention plate 66 is movable within a predetermined range from the frame 61 but does not come off from the frame 61, as a result of two stoppers 63, which are, for example, flanged screws, being threadedly attached to the respective projection portions 61a and thereby the backlash prevention plate 66 being brought into contact with the two stoppers 63 (see Fig. 22).

Note that the backlash prevention plate 66 here includes a screw hole 67, which is a thread connection portion to which an end portion of the front water feeding opening fitting 41 is threadedly connected.

The front water feeding opening fitting 41 is inserted and fitted in a hole portion 68 of the connector case 23 and threadedly attached to the screw hole 67 of the backlash prevention plate 66 with the end portion of the front water feeding opening fitting 41 fitted in the conduit block 70. Here, also, when the front water feeding opening fitting 41 and the backlash prevention plate 66 are threadedly connected, the backlash prevention plate 66 loosely fitted in the frame 61 rises and hits the stoppers 63. Then, the front water feeding opening fitting 41 is fixed via the frame 61 and the conduit block 70. At this time, the conduit block 70 is pressed by the front water feeding opening fitting 41 toward the frame 61, and a bottom face of the conduit block 70 hits a surface of the frame 61 and thereby fixed.

Note that in the front water feeding opening fitting 41, a seal 41a such as an O ring for holding airtightness when the front water feeding opening fitting 41 is attached to the hole portion 68 of the connector case 23 is provided. Also, in the conduit block 70, a seal 65a such as an O ring for maintaining airtightness between the front water feeding opening fitting 41 and the conduit block 70 is provided.

As described above, in the present reference example, also, even if manufacturing variations and/or deformations of components occur at a position of the hole portion 68 of the connector case 23, the backlash prevention plate 66 attached in a loosely fitted state to the frame 61 moves to a proper position, whereby misalignment between a center axis of the front water feeding opening fitting 41 and a center axis of the hole portion 68 can be suppressed, ensuring reliable maintenance of the airtightness of the connector case 23 and enabling the front water feeding opening fitting 41 to be stably fixed to the frame 61 via the conduit block 70.

### (Third reference example)

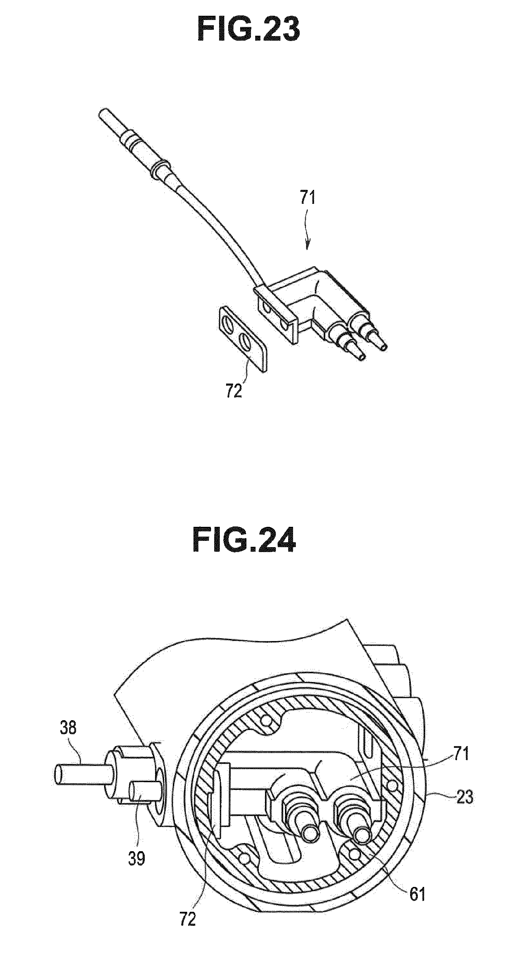

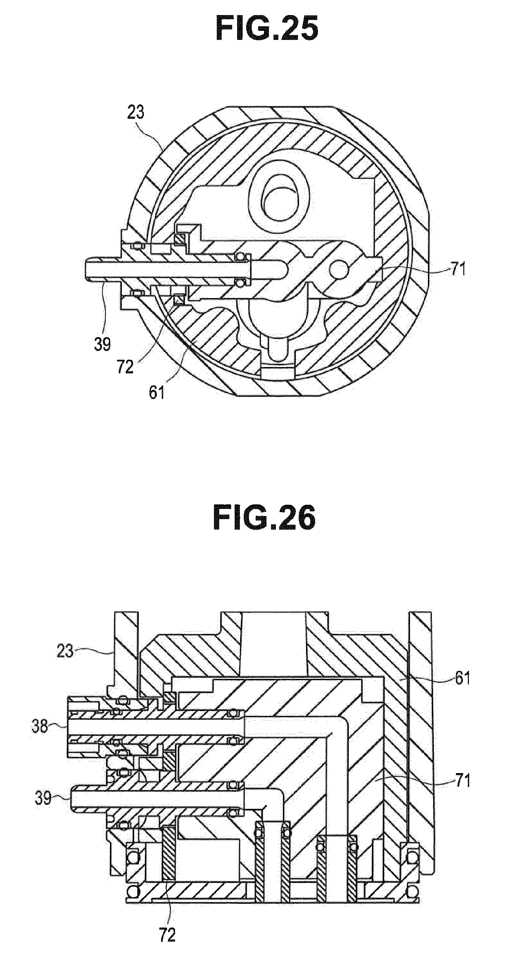

Figs. 23 to 26 relate to a third reference example: Fig. 23 is a perspective view illustrating an air/water feeding block with air/water feeding conduits incorporated therein; Fig. 24 is a cross-sectional view of a light source plug, which illustrates an attachment configuration of a water feeding opening fitting and an air feeding opening fitting provided at a connector case and the air/water feeding block provided inside; Fig. 25 is a transverse sectional diagram illustrating an attachment configuration of the water feeding opening fitting and the air feeding opening fitting provided at the connector case and the air/water feeding block provided inside; and Fig. 26 is a vertical sectional diagram illustrating an attachment configuration of the water feeding opening fitting and the air feeding opening fitting provided at the connector case and the air/water feeding block provided inside.

As illustrated in Figs. 23 to 26, here, as in the first and second reference examples, for attachment of the water feeding opening fitting 38 and the air feeding opening fitting 39, the water feeding opening fitting 38 and the air feeding opening fitting 39 are fixed via the frame 61, which is a reinforcing member, an air/water feeding block 71 (see Fig. 23) and a backlash prevention plate 72 inside the connector case 23.

The water feeding opening fitting 38 and the air feeding opening fitting 39 are inserted and fitted in the connector case 23 and the frame 61 disposed inside the connector case 23, and respective end portions of the water feeding opening fitting 38 and the air feeding opening fitting 39 are fitted in the air/water feeding block 71 inside the frame 61. The backlash prevention plate 72 is provided between the water feeding opening fitting 38 and the air feeding opening fitting 39, and the air/water feeding block 71, and the water feeding opening fitting 38 and the air feeding opening fitting 39 are threadedly connected to the backlash prevention plate 72. Note that the backlash prevention plate 72 is provided so as to be in contact with an inner face of the frame 61.

Here, when the water feeding opening fitting 38 and the air feeding opening fitting 39 are threadedly connected to the backlash prevention plate 72, the end faces of the water feeding opening fitting 38 and the air feeding opening fitting 39 hit the air/water feeding block 71 in which respective end faces of the water feeding opening fitting 38 and the air feeding opening fitting 39 are to be inserted and fitted, and a surface of the air/water feeding block 71 hits one of inner surfaces of the frame 61, and the backlash prevention plate 72 hits another surface on the side opposite to the one surface of the frame 61. In other words, if the amount of thread connection of the water feeding opening fitting 38 and the air feeding opening fitting 39 with respect to the backlash prevention plate 72 is increased, the backlash prevention plate 72 and the air/water feeding block 71 move in directions away from each other, and respective surfaces of the backlash prevention plate 72 and the air/water feeding block 71 hit the opposing inner faces of the frame 61, whereby the water feeding opening fitting 38 and the air feeding opening fitting 39 are fixed. Note that the airtightness between the water feeding opening fitting 38 and the air feeding opening fitting 39 fixed as described above and the connector case 23 and the air/water feeding block 71 are maintained by seals such as O rings.

From the above description, in the present reference example, also, misalignment between respective center axes of the water feeding opening fitting 38 and the air feeding opening fitting 39 and center axes of respective hole portions provided in the connector case 23, through which the water feeding opening fitting 38 and the air feeding opening fitting 39 are inserted, can be suppressed by the backlash prevention plate 72, ensuring reliable maintenance of the airtightness of the connector case 23 and enabling the water feeding opening fitting 38 and the air feeding opening fitting 39 to be stably fixed at respective predetermined positions in the connector case 23 of the light source plug 20.

The invention described in the above embodiment is not limited to the embodiment and the modifications thereof, and in practice, various modifications are possible without departing from the spirit of the invention. Furthermore, the above embodiment includes various phases of the invention, each of which can be extracted by a proper combination of a plurality of disclosed elements.

For example, even if some components are deleted from all the components indicated in the embodiment, a configuration with these components deleted can be extracted as the invention if the configuration can solve the indicated problem and provide the indicated effects.

The present application is filed claiming the priority of Japanese Patent Application No. 2011-260774 filed in Japan on November 29, 2011, the disclosure of which is hereby incorporated in the description, the claims and the drawings of the specification of the present application by reference.

## Claims

1. An endoscope comprising:
a tubular frame disposed on a wiring route of a cable, the frame allowing the cable to be inserted therethrough; and
a hole formed in a barrel portion of the frame, the hole allowing the cable to be inserted therethrough,
wherein an excess of the cable is drawn out from the hole and the excess is wound and stored on the barrel portion of the frame.

2. The endoscope according to claim 1, wherein the frame includes a circumferential groove at an outer circumference and the excess of the cable is wound and stored on a part of the barrel portion that forms a bottom face of the circumferential groove.

3. The endoscope according to claim 2, wherein the hole is formed in the part of the barrel portion that forms the bottom face of the circumferential groove.

4. The endoscope according to claim 3, wherein the excess of the cable is drawn out in a loop from the hole of the barrel portion and wound and stored on the barrel portion with the excess folded back in two.

5. The endoscope according to claim 4, wherein the frame has a circular tube shape and the hole is a long hole.

6. The endoscope according to any one of claims 1 to 5, comprising a fixing member that fixes the excess of the cable wound and stored on the barrel portion.

7. The endoscope according to any one of claims 1 to 6, wherein the cable includes one end connected to an image pickup apparatus and the other end connected to an electric connector that is attached/detached to/from a peripheral.

8. The endoscope according to any one of claims 1 to 7, comprising a rigid tube that covers the barrel portion of the frame with the excess of the cable wound and stored thereon.

9. The endoscope according to claim 8, comprising a cover that covers the rigid tube so as to reduce concentration of stress from an external force.
